(19) Europäisches Patentamt
European Patent Office
Office européen des brevets

(11) EP 3 895 226 B1

(12) EUROPEAN PATENT SPECIFICATION

(45) Date of publication and mention of the grant of the patent:
**25.10.2023 Bulletin 2023/43**

(21) Application number: **19817368.4**

(22) Date of filing: **16.12.2019**

(51) International Patent Classification (IPC):
***C07D 251/24*** *(2006.01)* ***C07C 13/00*** *(2006.01)*
***C07C 15/00*** *(2006.01)* ***H10K 85/60*** *(2023.01)*
***H10K 50/16*** *(2023.01)*

(52) Cooperative Patent Classification (CPC):
**C07D 251/24; C07D 401/10; C07D 405/04; C07D 409/04; C07F 9/6521; H10K 50/166; H10K 85/615; H10K 85/654; H10K 85/6574; H10K 85/6576;** H10K 50/165

(86) International application number:
**PCT/EP2019/085284**

(87) International publication number:
**WO 2020/120794 (18.06.2020 Gazette 2020/25)**

(54) **ORGANIC LIGHT EMITTING DEVICE AND A COMPOUND FOR USE THEREIN**

ORGANISCHE LICHTEMITTIERENDE VORRICHTUNG UND EINE VERBINDUNG ZUR VERWENDUNG DARIN

DISPOSITIF ÉLECTROLUMINESCENT ORGANIQUE ET COMPOSÉ À UTILISER EN SON SEIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.12.2018 EP 18212605**

(43) Date of publication of application:
**20.10.2021 Bulletin 2021/42**

(73) Proprietor: **Novaled GmbH**
**01099 Dresden (DE)**

(72) Inventors:
- **CARDINALI, Francois**
**01099 Dresden (DE)**
- **SCHOLZ, Johannes**
**01099 Dresden (DE)**
- **SCHULZE, Benjamin**
**01099 Dresden (DE)**
- **DENKER, Ulrich**
**01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

(56) References cited:
**EP-A1- 3 373 353**
**CN-A- 103 570 629**
**KR-A- 20180 063 709**
**KR-A- 20180 063 710**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

BACKGROUND ART

Organic light-emitting diodes (OLEDs), which are self-emitting devices, have a wide viewing angle, excellent contrast, quick response, high brightness, excellent driving voltage characteristics, and color reproduction. A typical OLED includes an anode, a hole transport layer (HTL), an emission layer (EML), an electron transport layer (ETL), and a cathode, which are sequentially stacked on a substrate. In this regard, the HTL, the EML, and the ETL are thin films formed essentially by organic and / or organometallic compounds.

When a voltage is applied to the anode and the cathode, holes injected from the anode electrode move to the EML, via the HTL, and electrons injected from the cathode electrode move to the EML, via the ETL. The holes and electrons mainly recombine in the EML to generate excitons. When the excitons drop from an excited state to a ground state, light is emitted. The injection and flow of holes and electrons should be balanced, so that an OLED having the above-described structure has excellent efficiency.

WO 2018/139662 A and EP3373353 A disclose an organic electroluminescent element including a light emitting layer, a first electron transport layer and a second electron transport layer arranged between an anode and a cathode, wherein the first electron transport layer and the second electron transport layer differ from each other.

Nevertheless, there is still a need to improve the performance of top emission and bottom emission OLEDs, in particular with respect to operating voltage and efficinecy thereof.

It is, therefore, the object of the present invention to provide novel organic light emitting devices and compounds for use therein overcoming drawbacks of the prior art, in particular compounds suitable to improve the performance of top emission and bottom emission OLEDs with respect to the operating voltage and efficincy thereof.

The above object is achieved by an organic light emitting device comprising a cathode, an anode, a light emitting layer, at least one first electron transport layer and at least one second electron transport layer, wherein the light emitting layer, the first electron transport layer and the second electron transport layer are arranged between the cathode and the anode, wherein the first electron transport layer comprises a compound of formula (I)

L -M

wherein in formula (I) L is selected from the $C_6$ to $C_{60}$ unsubstituted or substituted aryl, wherein the one or more substituents, if present, are independently selected from $C_2$ to $C_{24}$ alkenyl, $C_1$ to $C_{20}$ alkyl, $C_7$ to $C_{32}$ aryl-alkyl, $C_7$ to $C_{32}$ aryl-alkenyl; M is substituted pyrimidine or substituted triazine, wherein the one or more substituents are independently selected from $C_6$ to $C_{24}$ aryl wherein the $C_6$ to $C_{24}$ aryl may be unsubstituted or substituted with $C_1$ to $C_3$ alkyl; L and M may be connected via a direct bond or via a spacer moiety selected from non-condensed $C_6$ to $C_{18}$ aryl which may be unsubstituted or substituted wherein the one or more substituents, if present, are independently selected from $C_6$ to $C_{12}$ aryl, $C_2$ to $C_{20}$ heteroaryl, and phosphine oxide group; and the compound of formula (I) has a molecular dipole moment of ≤ 5 Debye; wherein the second electron transport layer comprises a compound of formula (II)

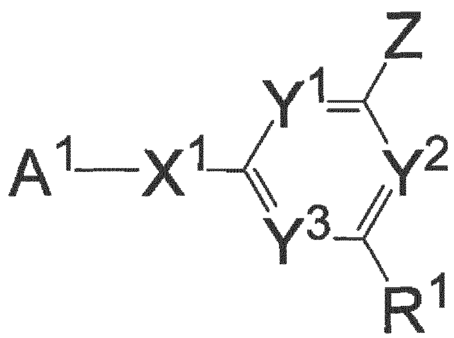

wherein in formula (II) $A^1$ is selected from unsubstituted or substituted $C_{10}$ to $C_{24}$ aryl comprising at least one aromatic ring system comprising at least two condensed 6-membered aromatic rings, wherein the one or more substituents, if present, are independently selected from $C_6$ to $C_{24}$ aryl, $C_2$ to $C_{20}$ heteroaryl and these substituents may be further substituted with a phosphine oxide group; $X^1$ is selected from unsubstituted or substituted $C_6$ to $C_{24}$ aryl, wherein the one or more substituents, if present, are independently selected from $C_6$ to $C_{12}$ aryl, $C_2$ to $C_{10}$ heteroaryl and these substituents may be further substituted with a phosphine oxide group or CN; $Y^1$ to $Y^3$ are independently selected from N and $CR^2$ provided that at least two of $Y^1$ to $Y^3$ are N, wherein the $R^2$ is selected from H or $C_1$ to $C_3$ alkyl; Z is selected from unsubstituted or substituted $C_6$ to $C_{24}$ aryl or unsubstituted or substituted $C_8$ to $C_{24}$ heteroaryl, wherein the one or

more substituents, if present, are independently selected from $C_6$ to $C_{24}$ aryl, $C_1$ to $C_{20}$ alkyl, phosphine oxide group and CN; and $R^1$ is selected from unsubstituted or substituted $C_6$ to $C_{24}$ aryl, wherein the one or more substituents, if present, are independently selected from $C_6$ to $C_{24}$ aryl, $C_1$ to $G_{20}$ alkyl, phosphine oxide group and CN.

It may be provided that the first electron transport layer does not comprise a dopant or an additive and that at the same time the second electron transport comprising the compound of formula (II) does, in addition to the compound of formula (II), comprises a dopant or an additive.

In this regard, suitable dopants or additives may be n-type dopants or n-type additives, such as LiQ, rare earth metals, alkali metals, alkaline earth metals, borates (CAS 14728-62-2), phenolates (such as CAS 1440864-50-5), phosphine imines (such as CAS 51870-56-5), guanidines (such as CAS 1623748-16-2), metal complexes as disclosed in US2009212280, heterocyclic compounds disclosed in US2007252140 etc.

In an organic electronic device, which may be an organic light emitting diode, the second electron transport layer may comprise at least one second compound besides the compound of formula (II). In this regard, it may be provided that the at least one second compound is a metal, a metal salt, a metal complex, a heterocyclic compound disclosed in US2007252140, an organic or metal-organic emitter compound or a mixture thereof. The metal complex may be an organic alkali metal complex or a complex disclosed in US2009212280. The organic alkali metal complex may be an alkali borate and/or phenolate and/or 8-hydroxyquinolinolato lithium.

The object is further achieved by a display device comprising the organic light emitting device as defined herein.

The object is further achieved by a lighting device comprising the organic light emitting device as defined herein.

It was suprisingly found by the inventors that organic light emitting devices comprising two different electron transport layers, respectively comprising the inventive compounds of formula (I) and (II) show improved performance specifically with regard to operating voltage and efficiency.

In this regard, it may be provided that the compound of formula (I) comprised in the first electron transport layer is different from the compound of formula (II) comprised in the second electron transport layer.

As used herein, the term "substituted with" means that a hydrogen atom of the respective group is replaced by another group, the so-called substituent.

In terms of the present application a fluorenylene or fluorenyl group is considered as a "non-condensed aryl".

It may be provided that the compound of formula (I) does not comprise a CN-group.

Two aromatic rings are considered to be condensed in terms of the present disclosure if the two aromatic rings share with each other two carbon atoms.

The term "condensed rings" describes a molecular structure in which two or more aromatic rings have two carbon atoms in common. For example, a napthyl group, an anthracene group etc. comprise condensed rings and are considered as condensed ring systems.

With respect to the inventive organic light emitting device it may be provided that $X^1$ is free of any condensed 6-membered aromatic rings.

In the inventive organic light emitting device L may be unsubstituted or substituted fluorene or phenylene, the phenylene being substituted with 1 to 5 phenyl groups, alternatively 3 to 5 phenyl groups, alternatively 4 phenyl groups. In this way, it is possible to further improve the perfomance of the organic light emitting device with respect to operating voltage and/or efficiency. In case L comprises condensed aromatic rings the compound of formula (I) is different from the compound of formula (II).

M may be substituted triazine wherein the one or more substituents, if present, are independently selected from $C_6$ to $C_{24}$ aryl which may be unsubstituted or substituted with $C_1$ to $C_3$ alkyl. In this way, it is possible to further improve the perfomance of the organic light emitting device with respect to operating voltage and/or efficiency.

The spacer moiety which may be present to connect L and M may be phenylene or biphenylene. In this way, it is possible to further improve the perfomance of the organic light emitting device with respect to operating voltage and/or efficiency.

In an embodiment L may comprise one or more $sp^3$-hybridized carbon atoms. The one or more $sp^3$-hybridized carbon atoms may act as a bridge-head atom between aromatic rings in group L. The aromatic rings bridged in this way are non-conjugated.

In a further embodiment L may comprise a fluorene moiety.

In another embodiment L may comprise a carbon-carbon double bond which is attached to an aryl or heteroaryl ring system via a direct bond.

In a further embodiment L comprises 3 to 10 unsubstituted phenyl or phenylene groups.

In an embodiment L comprises a system of interlinked but non-conjugated rings.

In a further embodiment L may comprise two or more conjugated 6-membered aromatic rings, preferrably three conjugated 6-membered aromatic rings.

In any case and in all embodiments according to the present invention the compound of formula (I) comprised in or constituting the first electron transport layer is different from the compound of formula (II) comprised in the second electron transport layer.

A1 may be unsubstituted or substituted anthracenyl or naphtyl, wherein wherein the one or more substituents are independently selected from $C_6$ to $C_{24}$ aryl, $C_2$ to $C_{20}$ heteroaryl and these substituents may be further substituted by a phosphine oxide group or CN. In this way, it is possible to further improve the perfomance of the organic light emitting device with respect to operating voltage and/or efficiency.

The group $X^1$ may be connected to the 9-position of the anthracene, the anthracene may comprise one substituent and the one substituent may be connected to the anthracene in the 10-position thereof. In this way, it is possible to further improve the perfomance of the organic light emitting device with respect to operating voltage and/or efficiency.

In the organic light emitting device the $X^1$ may be phenylene. In this way, it is possible to further improve the perfomance of the organic light emitting device with respect to operating voltage and/or efficiency.

It may be provided that all of the groups $Y^1$, $Y^2$ and $Y^3$ are N. In this way, it is possible to further improve the perfomance of the organic light emitting device with respect to operating voltage and/or efficiency.

Furthermore, it may be provided that Z is unsubstituted $C_8$ to $C_{24}$ heteroaryl. In this way, it is possible to further improve the perfomance of the organic light emitting device with respect to operating voltage and/or efficiency.

Furthermore, Z may be selected from unsubstituted or substituted $C_6$ to $C_{24}$ aryl. In this way, it is possible to further improve the perfomance of the organic light emitting device with respect to operating voltage and/or efficiency.

In another embodiment Z and $R^1$ may be selected the same. In this way, it is possible to further improve the perfomance of the organic light emitting device with respect to operating voltage and/or efficiency. Z and R1 may both be phenyl.

In the organic light emitting device, it may be provided that the at least one second electron transport layer comprises a metal, a metal salt or a metal complex. In this way, it is possible to further improve the perfomance of the organic light emitting device with respect to operating voltage and/or efficiency.

Alternatively, it may be provided that the second electron transport layer consists of one or more compound(s) having the formula (II) and/or (III).

The object is further achieved by a compound having the general formula (III) according to the claims.

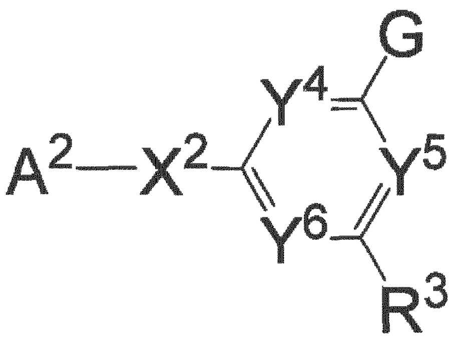

wherein $A^2$ is substituted anthracene or substituted $C_{10}$ aryl comprising at least two condensed 6-membered aromatic rings, wherein the one or more substituents are independently selected from $C_6$ to $C_{24}$ aryl and $C_2$ to $C_{20}$ heteroaryl and these substituents may be further substituted with a phosphine oxide group or CN; $X^2$ is phenylene; $Y^4$ to $Y^6$ are independently selected from N and $CR^4$ provided that at least two of $Y^4$ to $Y^6$ are N, wherein $R^4$ is selected from H or $C_1$ to $C_3$ alkyl; G is selected from unsubstituted or substituted $C_8$ to $C_{24}$ heteroaryl comprising at least one 5 -membered ring, wherein the one or more substituents, if present, are independently selected from $C_6$ to $C_{24}$ aryl, $C_l$ to $C_{20}$ alkyl, phosphine oxide group and CN and the heteroatom is selected from O, S, Se, N, alternatively O, S, Se; and $R^3$ is selected from unsubstituted or substituted $C_6$ to $C_{24}$ aryl, wherein the substituents are selected from $C_6$ to $C_{24}$ aryl, $C_1$ to $C_{20}$ alkyl, phosphine oxide group and CN.

In the compound of formula (III) $A^2$ is a substituted anthracene. In this way, it is possible to further improve the perfomance of the organic light emitting device with respect to operating voltage and/or efficiency.

The group $X^2$ may be connected to the 9-position of the anthracene. The anthracene may comprise one substituent and the one substituent is connected to the anthracene in the 10-position thereof. In this way, it is possible to further improve the perfomance of the organic light emitting device with respect to operating voltage and/or efficiency.

In the compound of formula (III) $X^2$ is phenylene. In this way, it is possible to further improve the perfomance of the organic light emitting device with respect to operating voltage and/or efficiency.

In the compound of formula (III) it may be provided that all of $Y^4$, $Y^5$ and $Y^6$ are N. In this way, it is possible to further improve the perfomance of the organic light emitting device with respect to operating voltage and/or efficiency.

G is selected from unsubstituted $C_8$ to $C_{24}$ heteroaryl comprising at least one 5-membered ring. In this way, it is possible to further improve the perfomance of the organic light emitting device with respect to operating voltage and/or

efficiency.

G may be selected from the group consisting of dibenzofurane, benzofurane, dibenzothiophene and benzothiophene. In this way, it is possible to further improve the perfomance of the organic light emitting device with respect to operating voltage and/or efficiency.

Furthermore, $R^3$ may be phenyl. In this way, it is possible to further improve the perfomance of the organic light emitting device with respect to operating voltage and/or efficiency.

In very preferred embodiments, the compound according to formula (I) has one of the following structures ET-1, ET-2, and ET-15.

| Compound Name | Structure |
|---|---|
| ET-1 | 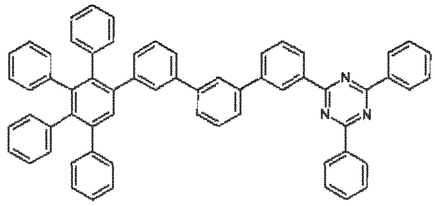 |
| ET-2 | 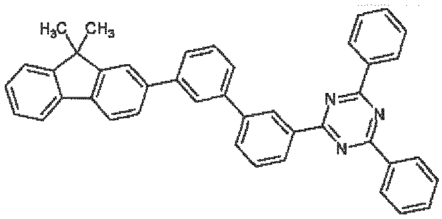 |
| ET-15 | 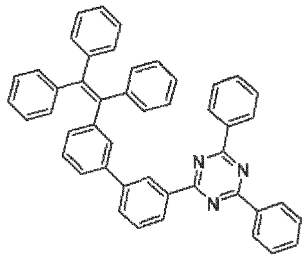 |

In a further preferred embodiment, the compound of formula (II) has one of the following structures ET-3, ET-11, ET-12, ET-13, ET14 and ET-16.

| Compound Name | Structure |
|---|---|
| ET-3 | 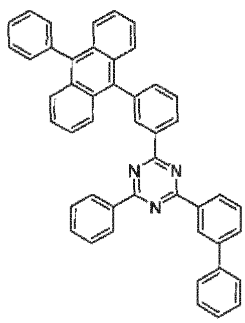 |

(continued)

| Compound Name | Structure |
|---|---|
| ET-11 | 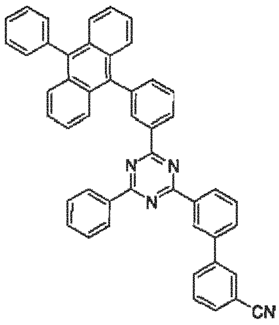 |
| ET-12 | 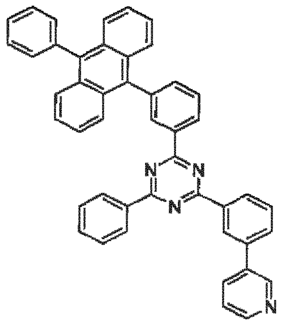 |
| ET-13 | 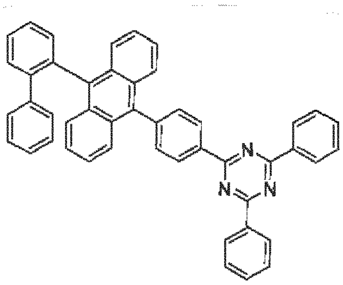 |
| ET-14 | 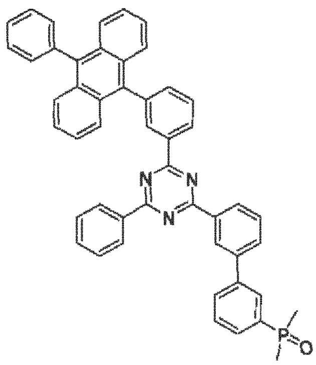 |

(continued)

| Compound Name | Structure |
|---|---|
| ET-16 | 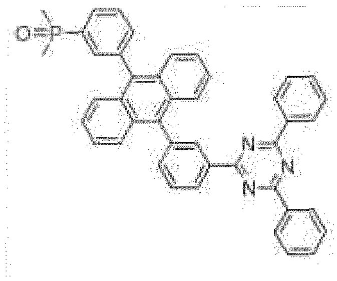 |

In very preferred embodiments, the compound of Formula (III) has one of the following structures ET-5 to ET-10 Compound ET4 is not forming part of the claimed invention.

| Compound Name | Structure |
|---|---|
| ET-4 | 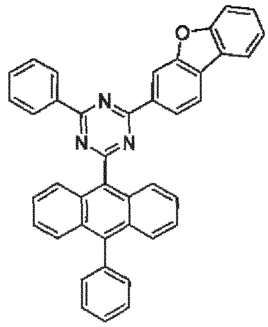 |
| ET-5 | 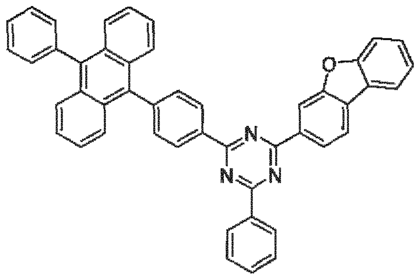 |
| ET-6 | 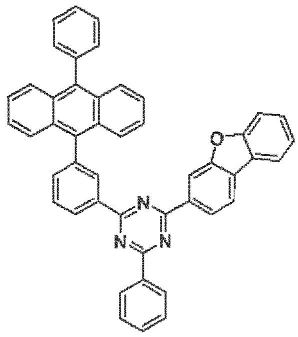 |

(continued)

| Compound Name | Structure |
|---|---|
| ET-7 | 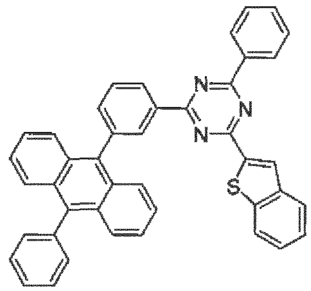 |
| ET-8 | 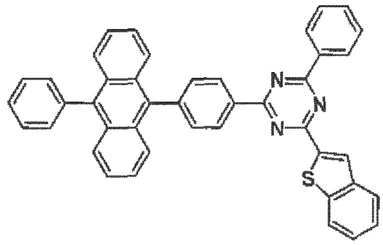 |
| ET-9 | 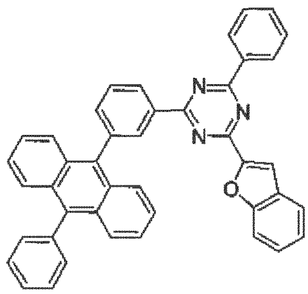 |
| ET-10 | 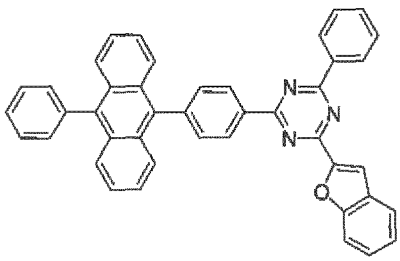 |

Further layers

In accordance with the invention, the organic light emitting device may comprise, besides the layers already mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

*Anode electrode*

Either a first electrode or a second electrode comprised in the inventive organic light emitting device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise, or consist of, a p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations may be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

The EML may be formed of a combination of host materials and emitter dopants. The EML may comprise a single host material or a plurality of host materials. The EML may comprise a single emitter dopant or a plurality of emitter dopants. Examples of the host materials are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

In case the EML comprises a plurality of host materials to form a host mixture the amount of each host material in the mixture of host materials may vary between 0.01 and 99.99 parts by weight.

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host or host mixture. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazine derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties,

without a substantial penalty in driving voltage.

The HBL may comprise a compound of formula (I) or the HBL may consist of a compound of formula (I).

The HBL may be the at least one first electron transport layer.

The HBL may be essentially free of n-type dopants or n-type additives.

The HBL may be in direct contact with the EML.

The HBL may be in direct contact with the ETL.

*Electron transport layer (ETL)*

The OLED according to the present invention comprises electron transport layers (ETL). In accordance with the invention, the at least one second electron transport layer is a layer comprising the compound represented by the general Formula (II) as defined above. Alternatively, the at least one second electron transport layer may comprise instead of or in addition to the compound of Formula (II) the compound of Formula (III) since Formula (III) is a special case of Formula (II) wherein the compounds of Formula (III) have further improved performance in the ETL of OLEDs in the inventive OLEDs.

In this context, it may be provided that the second electron transport layer comprises one or more of the compounds of Formula (II) and/or one or more of the compounds of Formula (III). In the case that the second electron transport layer comprises two different compounds, namely two compounds of formula (II) or two compounds of Formula (III) or one compound of Formula (II) and one compound of Formula (III), the mixing ratio, in weight % (wt%), maybe selected in the range of 1:1 to 1:9.The ETL may be the at least one second electron transport layer.

According to various embodiments the OLED may comprise further electron transport layers.

By suitably adjusting energy levels of particular layers of the ETL, the injection and transport of the electrons may be controlled, and the holes may be efficiently blocked. Thus, the OLED may have long lifetime.

The electron transport layer of the organic light emitting device may comprise the compound represented by general Formula (II) and Formula (III) as defined above as the organic electron transport matrix (ETM) material. The electron transport layer may comprise, besides the compound represented by the general Formulas (II) and (III), further ETM materials known in the art. Likewise, the electron transport layer may comprise as the only electron transport matrix material the compound represented by general Formulas (II) and (III).

In an embodiment the electron transport layer comprises two or more compounds represented by the general Formulas (II) and/or (III). In case that the inventive organic light emitting device comprises more than two electron transport layers, the compound represented by the general Formula (II) may be comprised in only one of the electron transport layers or in more than one of the electron transport layers. Likewise, it may be provided that the compound of Formula (III) is comprised in only one of the ETLs or in more than one of the ETLs. In all possible arrangements it is, of course, provided that the general requirements of the claims are met.

In accordance with the invention, the electron transport layer may comprise, besides the ETM material, at least one n-type dopant or n-type additive, as defined herein.

In another embodiment the electron transport layer is essentially free of n-type dopants or n-type additives.

"Essentially free" in this regard means that the respective compounds are only contained in the respective electron transport layer in amounts which cannot be avoided by common technical means. In this regard, it may be preferred that the first electron transport layer consists of one or more compound(s) of the formula (I) and/or the second electron transport layer consists of one or more compound(s) having the formula (II) and/or formula (III).

Further, the electron transport layer may comprise one or more n-type dopants or n-type additives. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be S-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (I) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

The n-type dopants or n-type additives are essentially non-emissive.

The ETL may be in direct contact with the EIL. In this regard, it may be preferred that the second electron transport layer is in direct contact with the EIL and the first electron transport layer is in direct contact with the second electron transport layer.

The ETL may be in direct contact with the cathode.

The ETL may be in direct contact with the CGL, preferably in direct contact with the n-type layer of the CGL. In this regard, it may be provided that the second electron transport layer is in direct contact with the n-type layer of the CGL and the first electron transport layer is in direct contact with the second electron transport layer.
The electron transport layer, preferably the first electron transport layer may be in direct contact with the emission layer.

*Electron injection layer (EIL)*

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, $Li_2O$, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may comprise a compound of formula (II). The EIL may comprise compound of formula (III).
The thickness of the EIL may be in the range from about o.1 nm to about 10 nm, for example, in the range from about 0.5 nm to about 9 nm. When the thickness of the EIL is within this range, the EIL may have satisfactory electron-injecting properties, without a substantial penalty in driving voltage.

*Cathode electrode*

The cathode electrode is formed on the EIL, if present. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.
The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed by a metal or metal alloy.
It is to be understood that the cathode electrode is not part of an electron injection layer or the electron transport layer.

*Charge generation layer*

The charge generation layer (CGL) may comprise a p- type and an n-type layer. An interlayer may be arranged between the p-type layer and the n-type layer.
Typically, the charge generation layer is a pn junction joining an n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.
Charge generating layers may be used in tandem devices, for example, in tandem OLEDs comprising, between two electrodes, two or more emission layers. In a tandem OLED comprising two emission layers, the n-type charge generation layer provides electrons for the first light emission layer arranged near the anode, while the hole generating layer provides holes to the second light emission layer arranged between the first emission layer and the cathode.
Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, FeCl3, FeF3, and SbCl5. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.
The n-type charge generation layer can be layer of a neat n-type dopant, for example of an electropositive metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds,

hydroxyquinoline derivatives like tris(8-hydroxyquinoline)aluminum, benzazole derivatives, and silole derivatives.

The hole generating layer may be arranged in direct contact to the n-type charge generation layer.

The n-type charge generation layer may comprise a compound of formula (II). The n-type charge generation layer may comprise compound of formula (III).

*Organic light-emitting diode (OLED)*

According to one aspect of the present invention, there is provided an organic light-emitting diode (OLED) comprising: a substrate; an anode electrode formed on the substrate; a hole injection layer, a hole transport layer, an emission layer, optionally a first electron transport layer comprising the compound of Formula (I) and a second electron transport layer comprising the compound of Formula (II) (respectively of Formula (III)) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, a first electron transport layer comprising the compound of Formula (I) and a second electron transport layer comprising the compound of Formula (II) (respectively of Formula (III)) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, a first electron transport layer comprising the compound of Formula (I) and a second electron transport layer comprising the compound of Formula (II) (respectively of Formula (III)), an electron injection layer, and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, a first electron transport layer comprising the compound of Formula (I) and a second electron transport layer comprising two compounds of Formula (II) (respectively of Formula (III)), an electron injection layer, and a cathode electrode.

According to various embodiments of the present invention, there may be provided OLEDs layers arranged between the above mentioned layers, on the substrate or on the top electrode.

According to one aspect, the OLED can comprise a layer structure of a substrate that is adjacently arranged to an anode electrode, the anode electrode is adjacently arranged to a first hole injection layer, the first hole injection layer is adjacently arranged to a first hole transport layer, the first hole transport layer is adjacently arranged to a first electron blocking layer, the first electron blocking layer is adjacently arranged to a first emission layer, the first emission layer is adjacently arranged to a first electron transport layer, the first electron transport layer is adjacently arranged to an n-type charge generation layer or, alternatively, adjacently arranged to a second electron transport layer which is arranged adjacently to the n-type charge generation layer, the n-type charge generation layer is adjacently arranged to a hole generating layer and an optional interlayer may be arranged between the p-type charge generation layer and the n-type charge generation layer, the hole generating layer is adjacently arranged to a second hole transport layer, the second hole transport layer is adjacently arranged to a second electron blocking layer, the second electron blocking layer is adjacently arranged to a second emission layer, between the second emission layer and the cathode electrode an optional electron transport layer and/or an optional injection layer are arranged.

An organic light-emitting diode (OLED) according to the invention may include an anode, a hole transport layer (HTL), an emission layer (EML), two electron transport layers (ETL) as defined herein, and a cathode, which are stacked on a substrate. In this regard, the HTL, the EML, and the ETL are thin films formed from organic compounds.

According to another aspect of the present invention, there is provided a method of manufacturing an organic light emitting device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

a first deposition source to release the compound of Formula (II) or (III) according to the invention, and

- a second deposition source to release a metal, a metal complex, an organo-metallic compound, a metal salt or an alkali or alkaline earth metal complex; alternatively an organic alkali or alkaline earth metal complex; alternatively 8-hydroxyquinolinolato lithium or alkali borate;

the method comprising the steps of forming the second electron transport layer; whereby for an organic light-emitting diode (OLED):

- the second electron transport layer is formed by releasing the compound of Formula (II) or (III) according to the invention from the first deposition source and a metal, a metal complex, an organo-metallic compound, a metal salt or an alkali or alkaline earth metal complex; alternatively an organic alkali or alkaline earth metal complex; alternatively 8-hydroxyquinolinolato lithium or alkali borate, from the second deposition source.

In another embodiment the second electron transport layer is formed by simultaneously releasing a compound of Formula (II) or (III) from a first deposition source and another compound of Formula (II) or (III) from a second deposition source.

According to various embodiments of the present invention, the method may further include forming on the anode electrode, an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, optionally a hole blocking layer is formed on the emission layer and an first or second electron transport layer is formed,
- and finally a cathode electrode is formed,
- optional a hole injection layer, a hole transport layer, and a hole blocking layer, formed in that order between the first anode electrode and the emission layer,
- optional an electron injection layer is formed between the electron transport layer and the cathode electrode.

According to various embodiments of the present invention, the method may further comprise forming an electron injection layer on the electron transport layer. However, according to various embodiments of the OLED of the present invention, the OLED may not comprise an electron injection layer.

According to various embodiments, the OLED may have the following layer structure, wherein the layers having the following order:

anode, hole injection layer, first hole transport layer, second hole transport layer, emission layer, optional hole blocking layer, the first electron transport layer, the second electron transport layer, optional electron injection layer, and cathode, or

anode, hole injection layer, first hole transport layer, second hole transport layer, the first electron transport layer, the second electron transport layer, optional hole blocking layer, optional electron injection layer, and cathode, or

anode, hole injection layer, first hole transport layer, second hole transport layer, emission layer, the first electron transport layer, the second electron transport layer, optional electron injection layer, and cathode.

In one embodiment, the organic electronic device according to the invention may further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be

selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

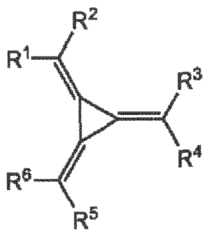 (XX) 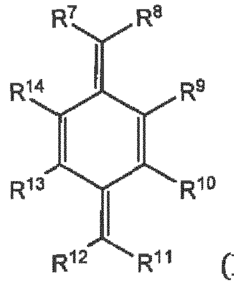 (XXIa) 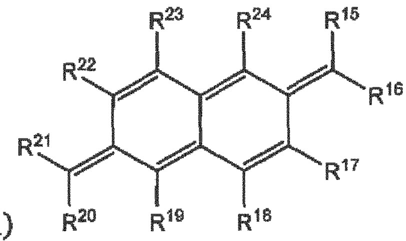 (XXIb),

wherein $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

Hereinafter, the embodiments are illustrated in more detail with reference to examples. However, the present disclosure is not limited to the following examples. Reference will now be made in detail to the exemplary aspects.

Details and definitions of the invention

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclo-hexyl.

The subscribed number n in C. relates to the total number of carbon atoms in the respective alkyl, arylene, heteroarylene or aryl group.

The term "aryl" or "arylene" as used herein shall encompass phenyl ($C_6$-aryl), fused aromatics, such as naphthalene, anthracene, phenanthracene, tetracene etc. Further encompassed are biphenyl and oligo- or polyphenyls, such as terphenyl etc.. Further encompassed shall be any further aromatic hydrocarbon substituents, such as fluorenyl etc. "Arylene" respectively "heteroarylene", referres to groups to which two further moieties are attached. In the present specification the term "aryl group" or "arylene group" may refer to a group comprising at least one hydrocarbon aromatic moiety, and all the elements of the hydrocarbon aromatic moiety may have p-orbitals which form conjugation, for example a phenyl group, a naphtyl group, an anthracenyl group, a phenanthrenyl group, a pyrenyl group, a fluorenyl group and the like. The aryl or arylene group may include a monocyclic or fused ring polycyclic (i.e., rings sharing adjacent pairs of carbon atoms) functional group.

The term "heteroaryl" as used herein refers to aryl groups in which at least one carbon atom is substituted with a heteroatom, preferably selected from N, O, S, B or Si.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazol, imidazole, oxazole, thiazole and the like.

The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably a heteroarylene ring may comprise at least 1 to 3 heteroatoms individually selected from N, S and/or O.

The term "heteroaryl" as used herewith shall encompass pyridine, quinoline, quinazoline, pyridine, triazine, benzimidazole, benzothiazole, benzo[4,5]thieno[3,2-d]pyrimidine, carbazole, xanthene, phenoxazine, benzoacridine, dibenzoacridine, dibenzofurane, benzofurane, dibenzothiophene, benzothiophene and the like.

In the present specification, the single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated",

In terms of the invention, a group is "substituted with" another group if one of the hydrogen atoms comprised in this group is replaced by another group, wherein the other group is the substituent.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

With respect to the inventive first and second electron transport layer layer as well as with respect to the inventive compound, the compounds mentioned in the experimental part are most preferred.

The inventive organic electronic device may be an organic electroluminescent device (OLED) an organic photovoltaic device (OPV), a lighting device, or an organic field-effect transistor (OFET). A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention may comprise more than one emission layer, preferably two or three emission layers. An OLED comprising more than one emission layer is also described as a tandem OLED or stacked OLED.

The organic electroluminescent device (OLED) may be a bottom- or top-emission device.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", "wt.-%", "percent by weight", "% by weight", parts by weight and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of", "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the electron transport layers comprising the compound of formula (I), formula (II) or formula (III) are

essentially non-emissive or non-emitting.

The operating voltage, also named U, is measured in Volt (V) at 10 milliAmpere per square centimeter (mA/cm2).

The candela per Ampere efficiency, also named cd/A efficiency is measured in candela per ampere at 10 milliampere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode electrode and cathode electrode may be described as anode electrode / cathode electrode or anode electrode / cathode electrode or anode electrode layer / cathode electrode layer.

Room temperature, also named ambient temperature, is 23° C.

BRIEF DESCRIPTION OF THE DRAWINGS

These and/or other aspects and advantages of the present invention will become apparent and more readily appreciated from the following description of the exemplary embodiments, taken in conjunction with the accompanying drawings, of which:

FIG. 1 is a schematic sectional view of a tandem OLED comprising a charge generation layer (CGL), according to an exemplary embodiment of the present invention.

DETAILED DESCRIPTION

Reference will now be made in detail to the exemplary embodiments of the present invention, examples of which are illustrated in the accompanying drawings, wherein like reference numerals refer to the like elements throughout. The exemplary embodiments are described below, in order to explain the aspects of the present invention, by referring to the figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

Referring to Fig. 1, the OLED 200 includes a substrate 110, an anode 120, a first hole injection layer (HIL) 130, a first hole transport layer (HTL) 140, a first electron blocking layer (EBL) 145, a first emission layer (EML) 150, a first electron transport layer or hole blocking layer (HBL) 155, a second electron transport layer 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a further first electron transport layer or hole blocking layer (HBL) 156, a further second electron transport layer 161, a second electron injection layer (EIL) 181 and a cathode 190.

While not shown in Fig. 1 a sealing layer may further be formed on the cathode electrodes 190, in order to seal the OLED 200. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

**Experimental Part**

The invention is furthermore illustrated by the following examples which are illustrative only and non-binding.

Example structures

| Compound Name | Structure | Respective Formula in terms of the claims |
|---|---|---|
| ET-1 | 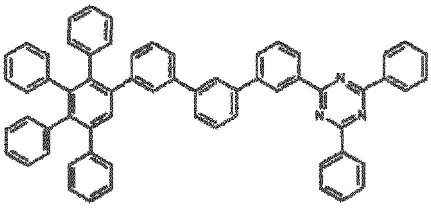 | Compound of formula (I) |
| ET-2 | 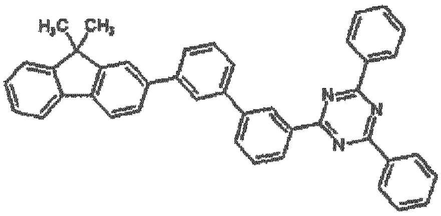 | Compound of formula (I) |
| ET-15 | 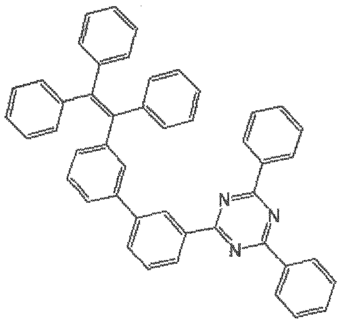 | Compound of formula (I) |
| ET-3 | 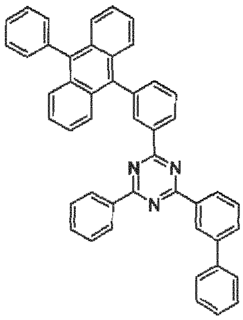 | Compound of formula (II) |
| ET-11 | 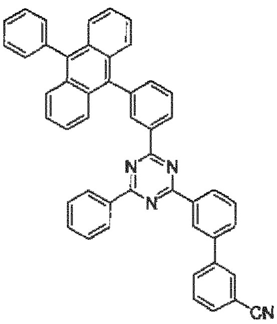 | Compound of formula (II) |

(continued)

| Compound Name | Structure | Respective Formula in terms of the claims |
|---|---|---|
| ET-12 | 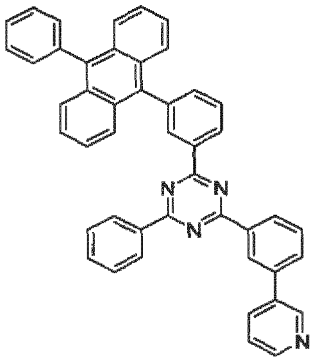 | Compound of formula (II) |
| ET-13 | 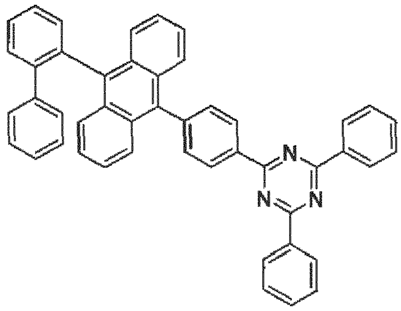 | Compound of formula (II) |
| ET-14 | 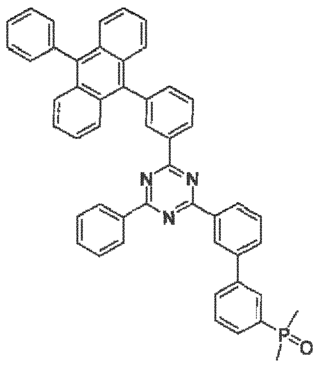 | Compound of formula (II) |
| ET-16 | 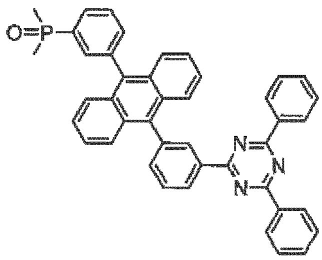 | Compound of formula (II) |

(continued)

| Compound Name | Structure | Respective Formula in terms of the claims |
|---|---|---|
| ET-4 | 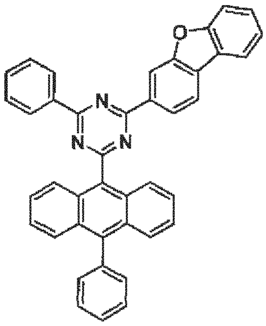 | Reference compound |
| ET-5 | 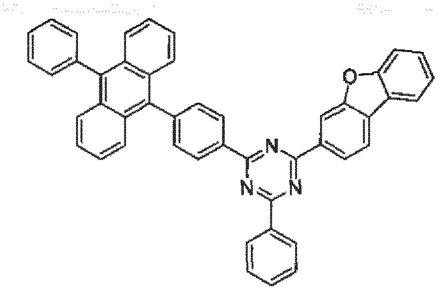 | Compound of formula (III) |
| ET-6 | 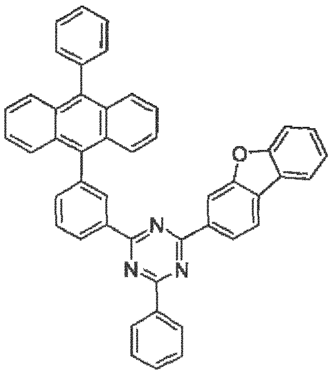 | Compound of formula (III) |
| ET-7 | 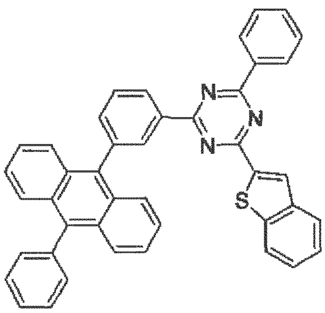 | Compound of formula (III) |
| ET-8 | 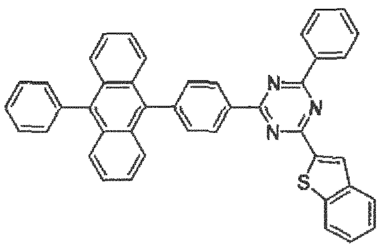 | Compound of formula (III) |

(continued)

| Compound Name | Structure | Respective Formula in terms of the claims |
|---|---|---|
| ET-9 | 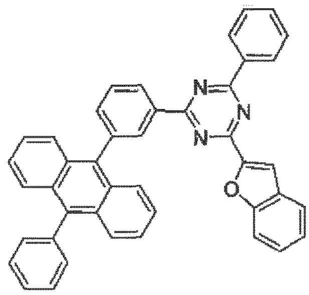 | Compound of formula (III) |
| ET-10 | 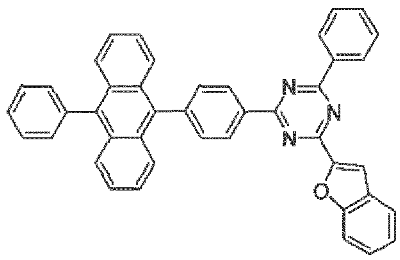 | Compound of formula (III) |

Experimental data

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Rate onset temperature

The rate onset temperature (TRO) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10-5 mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 oC. If the rate onset temperature is below 200 oC the evaporation may be too rapid and therefore difficult to control. If the rate onset temperature is above 255 oC the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Reduction potential

The reduction potential is determined by cyclic voltammetry with poteniostatic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard Fc+/Fc redox couple, afforded finally the values reported above. All studied compounds as well as the reported comparative compounds showed well-defined reversible electrochemical behaviour.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_1}$ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LLTMQ energy levels of the molecular structures are determined by applying the hybrid functional B3LYP with a 6-31G* basis set in the gas phase. If more than one conformation is viable, the conformation with the lowest total energy is selected.

Synthesis Procedures

General synthetic procedure for the preparation the coupling between unsymmetrically substituted chlorotriazine (A) and boronic acid (B):

**Synthesis of 2-(dibenzo[b,d]furan-3-yl)-4-phenyl-6-(10-phenylanthracen-9-yl)-1,3,5-triazine** reference compound ET-4 not forming part of the present invention

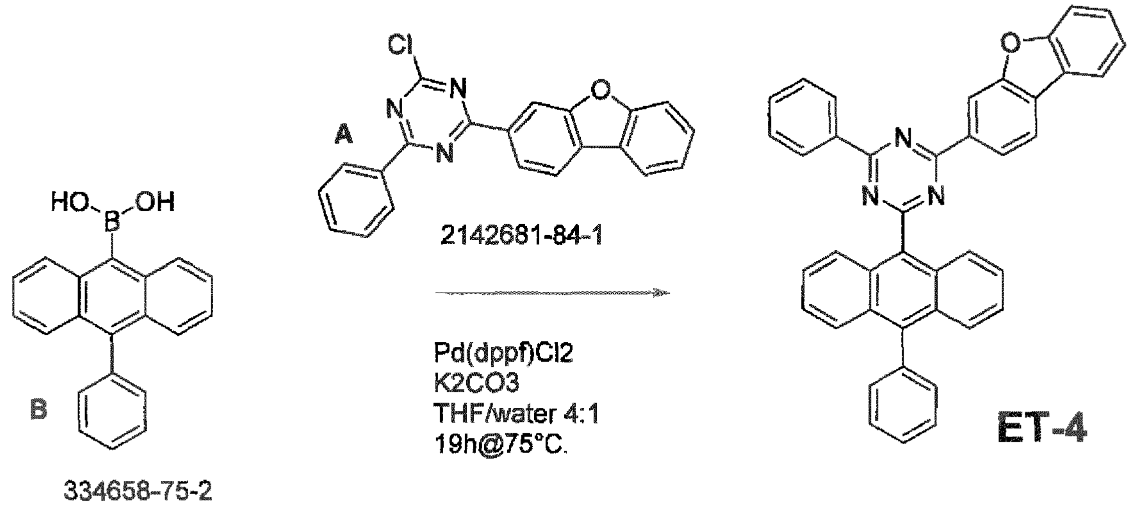


In a 500mL 3-necked flask, were placed following compounds: 2-chloro-4-(dibenzo[b,d]furan-3-yl)-6-phenyl-1,3,5-triazine (A, 10g, 28mmol, 1eq.) , phenylanthranylboronic acid (B, 9.2g, 31mmo, 1.1eq.) and potassium carbonate (7.7g, 55.9mmol, 2eq.) together with THF (224mL) and dest. water (56mL). The yellow suspension was degassed by bubbling N2 for 60 minutes. Then Pd(dppf)Cl2 (0.1g, 0.14mmol, 0.5%molar) was added under nitrogen counter flow. The reaction was then heated 19h at 75°C (bath temperature) under nitrogen. TLC control shows reaction completion. After cooling to rt and evaporation of the solvent, residue was extracted with 4L DCM/Chloroform and the organic layer washed in portions with in total 7.5L of water until pH neutral. After filtering over Florisil layer, the organic layer was evaporated and the residue treated by 200mL hexane. Precipitate was filtered and dried under high vacuum two times at 120°C (melting). Obtained 14.4g (89%). MS [M+H]+ 576.

According to this procedure, the synthesis of following molecules was performed:

**2-([1,1'-biphenyl]-3-yl)-4-phenyl-6-(3-(10-phenylanthracen-9-yl)phenyl)-1,3,5-triazine (ET-3)**

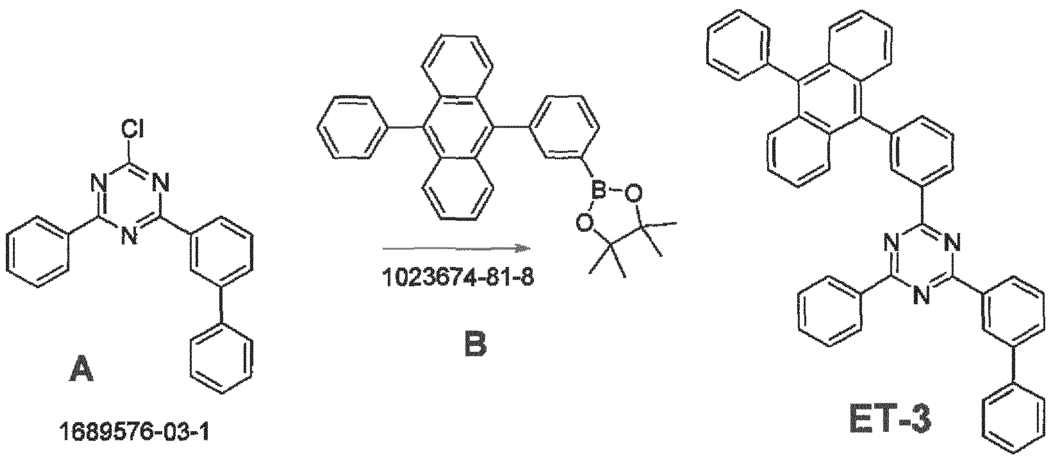


A: 15g 43.6mmol (1eq.) and B 17.9g 47.99mmol (1.1eq.). Obtained 20.3g (73%) MS [M+H]+ 638.

**2-(dibenzo[b,d]furan-3-yl)-4-phenyl-6-(3-(10-phenylanthracen-9-yl)phenyl)-1,3,5-triazine (ET-6)**

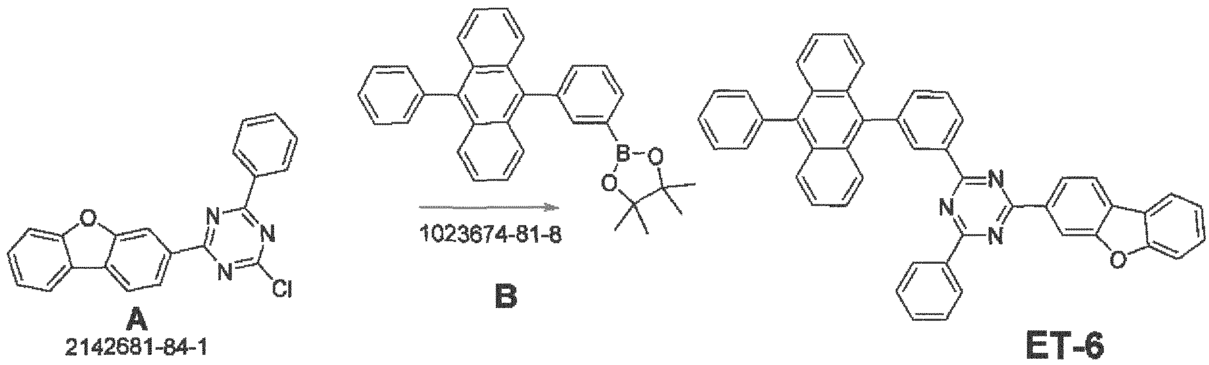


A: 7.91g 22.1mmol (1eq.) and B: 10.6g 23.23mmol (1.05eq.). Obtained 13.5g (94%). [M+H]+ 652.

**2-(dibenzo[b,d]furan-3-yl)-4-phenyl-6-(4-(10-phenylanthracen-9-yl)phenyl)-1,3,5-triazine**

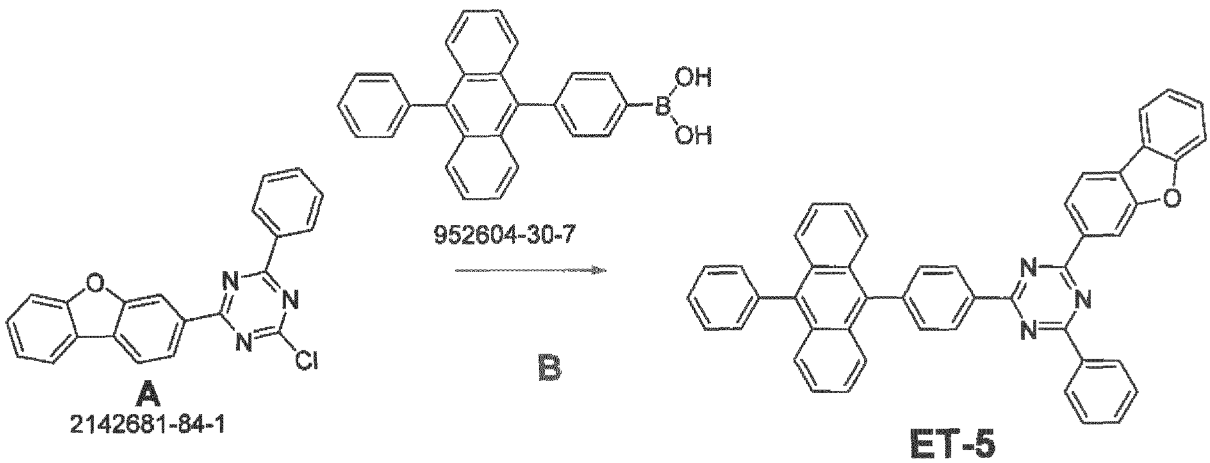


A: 8.14g 22.7mmol (1eq.) and B: 8.94g 23.9mmol (1.05eq.). Obtained 11.6g (78%) MS [M+H]+ 652.

**2-(benzo[b]thiophen-2-yl)-4-(4-chlorophenyl)-6-phenyl-1,3,5-triazine**

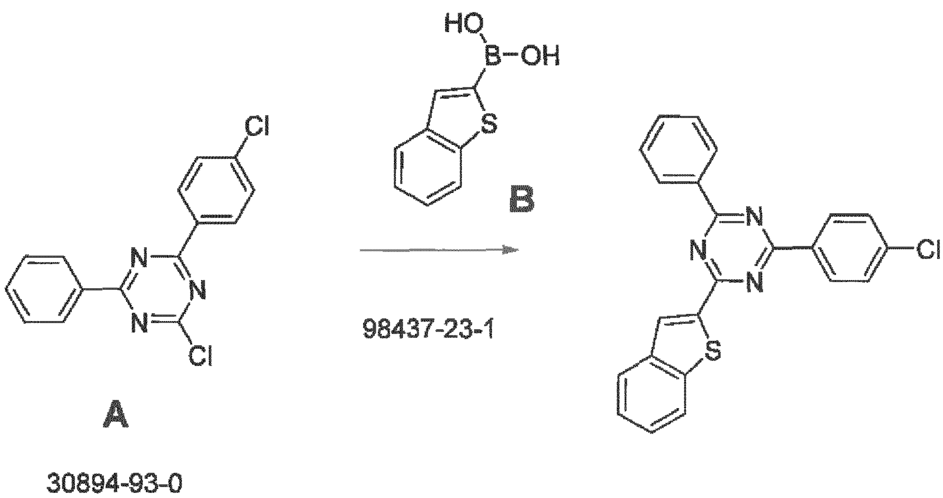

A: 50g 166mmol (1eq.) and B: 30.93g 174mmol (1.05eq.). Obtained 9.3g. Material was further reacted as follows:

**2-(benzo[b]thiophen-2-yl)-4-phenyl-6-(4-(10-phenylanthracen-9-yl)phenyl)-1,3,5-triazine**

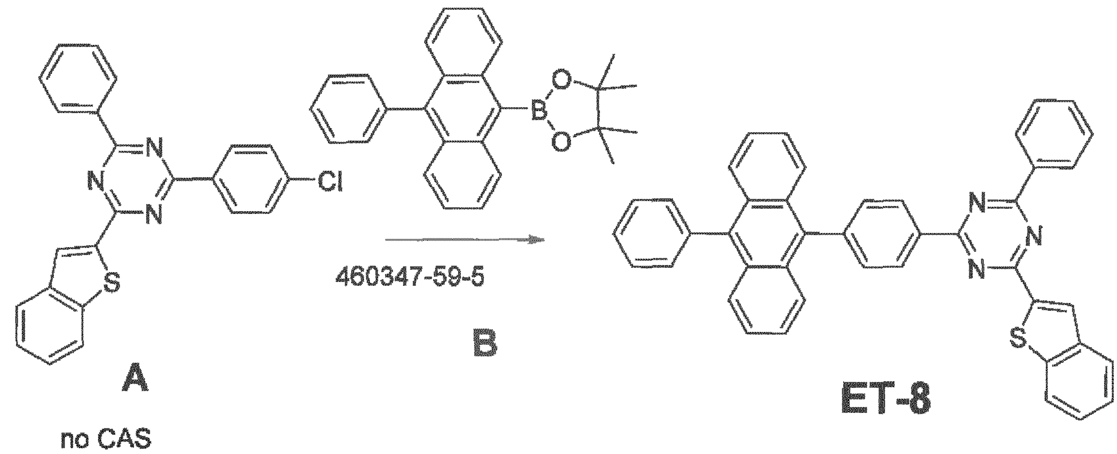


A

no CAS

B

ET-8

In a 1000mL 2-necked flask equipped with a mechanical stirrer, were placed following compounds: 2-(benzo[b]thiophen-2-yl)-4-(4-chlorophenyl)-6-phenyl-1,3,5-triazine (A, 9g, 22.5mmol, 1eq.) , 4,4,5,5-tetramethyl-2-(10-phenylanthracen-9-yl)-1,3,2-dioxaborolane (B, 10.3g, 27mmol, 1.2eq.) and dissolved with the addition of 225mL THF. In Parallel, an aqueous solution of potassium phosphate (9.6g, 45mmol, 2eq.) in 22.5mL distillated water was degassed by bubbling $N_2$ for 30 minutes. The base solution was transferred into the THF solution and the mixture degassed again. Then as catalyst Pd-172 [1798781-99-3] (0.27g, 0.45mmol, 2%molar) was added under nitrogen counter flow. The orange solution was then heated 18h at 55°C (bath temperature) under nitrogen. TLC control (Eluent DCM/hexane 1:1) shows total consumption of B. After cooling to rt, the precipitate was isolated by filtration, washed with 20mL THF and 20mL water. Dissolution in 1L of hot chloroform followed by hot filtration over Florisil pad, washed off with 3×100mL of hot chloroform afforded a bright yellow solution that was reduced to around 200mL and treated at room temperature with 200mL of hexane. The precipitate was isolated by filtration. Recrystallisation two times in 150mL then 2510mL followed by drying under vacuum overnight afforded 9.4g (67%) of product. ESI-MS [M+H]+ 618.

General procedure for fabrication of OLEDs

For the top emission OLEDs 1 to 3 a substrate with dimensions of 150 mm × 150 mm × 0.7 mm was ultrasonically cleaned with a 2% aquatic solution of Deconex FPD 211 for 7 minutes and then with pure water for 5 minutes, and dried for 15 minutes in a spin rinse dryer. Subsequently, Ag was deposited as anode at a pressure of 10-5 to 10-7 mbar.

Then, HT-1 and D-1 were vacuum co-deposited on the anode to form a HIL. Then, HT-1 was vacuum deposited on the HIL, to form an HTL. Then, HT-2 was vacuum deposited on the HTL to form an electron blocking layer (EBL).

Afterwards the emission layer was formed on the EBL by co-deposition of HOST-1 and EMITTER-1.

Then, the at least one first electron transport layer was vacuum deposited onto the emission layer to form the hole blocking layer (HBL). Then, the at least one second electron transport layer is formed on the hole blocking layer.

Then, the electron injection layer is formed on the electron transporting layer by deposing Yb.

Ag is evaporated at a rate of 0.01 to 1 Å/s at 10-7 mbar to form a cathode.

A cap layer of HT-1 is formed on the cathode.

OLED 1 comprises the compound ET-1 (compound of formula (I) ) in the at least one first electron transport layer and the compound ET-3 (compound of formula (II) ) in the at least one second electron transport layer. The details of the layer stack in the devices are given below. A slash "/" separates individual layers. Layer thicknesses are given in squared brackets [...], mixing ratios in wt% given in round brackets (...):

Layer stack details of OLED 1: silver [100 nm]/HT-1:D-1 (92:8 )[10 nm]/HT-1 [118 nm] / HT-2 [5 nm]/HOST-1:EMITTER-1 (97:3) [20 nm]/ET-1 [5 nm]/ET-3:LiQ (1:1) [31 nm]/Yb [2 nm]/silver:magnesium (9:1) [13 nm] / HT-1 [70 nm]

OLED 2 comprises the compound ET-2 (compound of formula (I) ) in the at least one first electron transport layer and the compound ET-3 (compound of formula (II) ) in the at least one second electron transport layer. The details of the layer stack in the devices are given below. A slash "/" separates individual layers. Layer thicknesses are given in squared brackets [...], mixing ratios in wt% given in round brackets (...):

Layer stack details of OLED 2: silver [100 nm]/HT-1:D-1 (92:8 )[10 nm]/HT-1 [118 nm] / HT-2 [5 nm]/HOST-1:EMITTER-1 (97:3) [20 nm]/ET-2 [5 nm]/ET-3:LiQ (1:1) [31 nm]/Yb [2 nm]/silver:magnesium (9:1) [13 nm] / HT-1 [70 nm]

OLED 3 a reference example not forming part of the present invention comprises the compound ET-1 (compound of formula (I) ) in the at least one first electron transport layer and the compound ET-4 (reference compound) in the at least one second electron transport layer. The details of the layer stack in the devices are given below. A slash "/" separates individual layers. Layer thicknesses are given in squared brackets [...], mixing ratios in wt% given in round brackets (...):

Layer stack details of OLED 3: silver [100 nm]/HT-1:D-1 (92:8 )[10 nm]/HT-1 [118 nm] / HT-2 [5 nm]/HOST-1:EMITTER-1 (97:3) [20 nm]/ET-1 [5 nm]/ET-4:LiQ (1:1) [31 nm]/Yb [2 nm]/silver:magnesium (9:1) [13 nm] / HT-1 [70 nm]

OLED 4 comprises the compound ET-1 (compound of formula (I) ) in the at least one first electron transport layer and the compound ET-16 (compound of formula (II) ) in the at least one second electron transport layer. The details of the layer stack in the devices are given below. A slash "/" separates individual layers. Layer thicknesses are given in squared brackets [...], mixing ratios in wt% given in round brackets (...):

Layer stack details of OLED 4: silver [100 nm]/HT-1:D-1 (92:8 )[10 nm]/HT-1 [118 nm] / HT-2 [5 nm]/HOST-1:EMITTER-1 (97:3) [20 nm]/ET-1 [5 nm]/ET-3:ET-16 (7:3) [31 nm]/Yb [2 nm]/silver:magnesium (9:1) [13 nm] / HT-1 [70 nm]

**Technical Effect of the invention**

The OLED devices according to the invention show high EQE efficiency at low voltage. Using the particularly preferred compounds of formula (III) for the at least one second electron transport layer further improves the EQE efficiency at a similar operating voltage.

List of compounds used

| | IUPAC name | Reference |
|---|---|---|
| HT-1 | N-([1,1'-biphenyl]-4-yl)-9,9-dimethyl-N-{4-(9-phenyl-9H-carbazol-3-yl)phenyl)-9H-fluoren-2-amine [CAS 1242056-42-3] | US2016322581 |
| HT-2 | N-(4-(dibenzo[b,d]furan-4-yl)phenyl)-N-(4-(9-phenyl-9H-fluoren-9-yl)phenyl)-[1,1'-biphenyl]-4-amine [CAS 1824678-59-2] | WO2015174640 |
| D-1 | 4.4'.4"-((1E,1'E,1"E)-cyclopropane-1,2,3-triyhdenetris (cyanomethanylylidene))tris(2,3,5,6-tetrafluorobenzonitrile) [CAS 1224447-88-4] | US2008265216 |
| HOST-1 | H09 (Fluorescent-blue host material) | Commercially available from Sun Fine Chemicals, Inc, S. Korea |
| EMITTER-1 | BD200 (Fluorescent-blue emitter material) | Commercially available from Sun Fine Chemicals, Inc, S. Korea |

(continued)

| | IUPAC name | Reference |
|---|---|---|
| ET-1 | 2,4-diphenyl-6-(4',5',6'-triphenyl-[1,1':2',1":3",1"':3"',1""-quinquephenyl]-3""-yl)-1,3,5-triazine [CAS 2032364-64-8] | WO2016171358 |
| ET-2 | 2-(3'-(9,9-dimethyl-9H-fluoren-2-yl)-[1,1'-biphenyl]-3-yl)-4,6-diphenyl-1,3,5-triazine [CAS 1955543-57-3] | WO2016105141 |
| ET-3 | 2-([1,1'-biphenyl]-3-yl)-4-phenyl-6-(3-(10-phenylanthracen-9-yl)phenyl)-1,3,5-triazine | |
| ET-4 | 2-(dibenzo[b,d]furan-3-yl)-4-phenyl-6-(10-phenylanthracen-9-yl)-1,3,5-triazine | |
| LiQ | 8-Hydroxyuinolinolato-lithium [CAS 850918-68-2] | WO2013079217 |

Table 1: Properties of compounds ET-1 to ET-4, both used in the at least one second electron transport layer

| | Dipole moment simulated by DFT (B3LYP_Gaussian / 6-31G*, gas phase) [Debye] | mp [0C] | Tg [0C] | TRO [0C] |
|---|---|---|---|---|
| ET-1 | 0.30 | - | 141 | 267 |
| ET-2 | 0.31 | 167 | 95 | 220 |
| ET-3 | 0.37 | 286 | 127 | 243 |
| ET-4 | 0.78 | 273 | 145 | 224 |
| ET-5 | 0.55 | 333 | 165 | 282 |
| ET-6 | 1.72 | 330 | 158 | 262 |
| ET-8 | 0.94 | 321 | 159 | 265 |

Table 2: Performance of an organic electroluminescent device comprising the compounds of formula (I) in the at least one first electron transport layer and compounds of formula (II) and formula (III) in the at least one second electron transport layer.

| OLED device examples | First electron transport layer | Second electron transport layer (wt ratio) | Operating voltage at 10 mA/cm$^2$ (V) | EQE at 10mA/cm$^2$ |
|---|---|---|---|---|
| OLED-1 | ET-1 | ET-3: LiQ (1:1) | 3.5 | 15.5 |
| OLED-2 | ET-2 | ET-3: LiQ (1:1) | 3.6 | 14.9 |
| OLED-3 Reference Example | ET-1 | ET-4 : LiQ (1:1) | 3.6 | 16.1 |
| OLED-4 | ET-1 | ET-3: ET-16 (7:3) | 3.5 | 15.4 |

## Claims

1. Organic light emitting device comprising a cathode (190), an anode (120), a light emitting layer (150, 151), at least one first electron transport layer and at least one second electron transport layer, wherein the light emitting layer, the first electron transport layer (156) and the second electron transport layer (161) are arranged between the

cathode and the anode,

wherein the first electron transport layer comprises a compound of formula (I)

L-M

wherein in formula (I)
L is selected from the $C_6$ to $C_{60}$ unsubstituted or substituted aryl, wherein the one or more substituents, if present, are independently selected from $C_2$ to $C_{24}$ alkenyl, $C_1$ to $C_{20}$ alkyl, $C_7$ to $C_{32}$ aryl-alkyl, $C_7$ to$C_{32}$ aryl-alkenyl;
M is substituted pyrimidine or substituted triazine, wherein the one or more substituents, are independently selected from $C_6$ to $C_{24}$ aryl wherein the $C_6$ to $C_{24}$ aryl may be unsubstituted or substituted with $C_1$ to $C_3$ alkyl;
L and M may be connected via a direct bond or via a spacer moiety selected from non-condensed $C_6$ to $C_{18}$ aryl which may be unsubstituted or substituted wherein the one or more substituents, if present, are independently selected from $C_6$ to $C_{12}$ aryl, $C_2$ to $C_{20}$ heteroaryl, and phosphine oxide group; and
the compound of formula (I) has a molecular dipole moment of ≤ 5 Debye; wherein the second electron transport layer comprises a compound of formula (II)

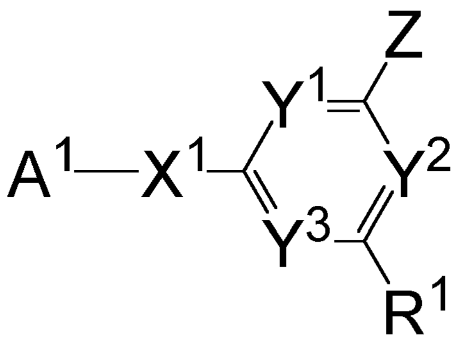

wherein in formula (II)
$A^1$ is selected from unsubstituted or substituted $C_{10}$ to $C_{24}$ aryl comprising at least one aromatic ring system comprising at least two condensed 6-membered aromatic rings, wherein the one or more substituents, if present, are independently selected from $C_6$ to $C_{24}$ aryl, $C_2$ to $C_{20}$ heteroaryl and these substituents may be further substituted with a phosphine oxide group;
$X^1$ is selected from unsubstituted or substituted $C_6$ to $C_{24}$ aryl, wherein the one or more substituents, if present, are independently selected from $C_6$ to $C_{12}$ aryl, $C_2$ to $C_{10}$ heteroaryl and these substituents may be further substituted with a phosphine oxide group or CN;
$Y^1$ to $Y^3$ are independently selected from N and $CR^2$ provided that at least two of $Y^1$ to $Y^3$ are N, wherein the $R^2$ is selected from H or $C_1$ to $C_3$ alkyl;
Z is selected from unsubstituted or substituted $C_6$ to $C_{24}$ aryl or unsubstituted or substituted $C_8$ to $C_{24}$ heteroaryl, wherein the one or more substituents, if present, are independently selected from $C_6$ to $C_{24}$ aryl, $C_1$ to $C_{20}$ alkyl, phosphine oxide group and CN; and
$R^1$ is selected from unsubstituted or substituted $C_6$ to $C_{24}$ aryl, wherein the one or more substituents, if present, are independently selected from $C_6$ to $C_{24}$ aryl, $C_1$ to $C_{20}$ alkyl, phosphine oxide group and CN.

2. Organic light emitting device according to claim 1, wherein L is unsubstituted or substituted fluorene or phenylene, the phenylene being substituted with 1 to 5 phenyl groups.

3. Organic light emitting device according to claim 1 or 2, wherein M is substituted triazine wherein the one or more substituents, are independently selected from $C_6$ to $C_{24}$ aryl which may be unsubstituted or substituted with $C_1$ to $C_3$ alkyl.

4. Organic light emitting device according to any of the preceding claims, wherein the space moiety is phenylene or biphenylene.

5. Organic light emitting device according to any of the preceding claims, wherein $A^1$ is substituted anthracene, wherein the one or more substituents, are independently selected from $C_6$ to $C_{24}$ aryl and $C_2$ to $C_{20}$ heteroaryl and these

substituents may be further substituted with a phosphine oxide group or CN.

**6.** Organic light emitting device according to any of the preceding claims, wherein $X^1$ is phenylene.

**7.** Organic light emitting device according to any of the preceding claims, wherein all $Y^1$ to $Y^3$ are N.

**8.** Organic light emitting device according to any of the preceding claims, wherein Z is selected from unsubstituted or substituted $C_6$ to $C_{24}$ aryl.

**9.** Organic light emitting device according to any of the preceding claims, wherein the at least one second electron transport layer comprises a metal, a metal salt or a metal complex.

**10.** Compound having the general formula (III)

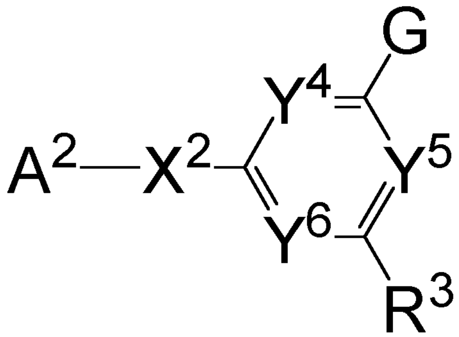

wherein

$A^2$ is substituted anthracene or substituted $C_{10}$ aryl comprising at least two condensed 6-membered aromatic rings, wherein the one or more substituents are independently selected from $C_6$ to $C_{24}$ aryl and $C_2$ to $C_{20}$ heteroaryl and these substituents may be further substituted with a phosphine oxide group or CN;
$X^2$ is phenylene;
$Y^4$ to $Y^6$ are independently selected from N and $CR^4$ provided that at least two of $Y^4$ to $Y^6$ are N, wherein $R^4$ is selected from H or $C_1$ to $C_3$ alkyl;
G is selected from unsubstituted or substituted $C_8$ to $C_{24}$ heteroaryl comprising at least one 5-membered ring, wherein the one or more substituents, if present, are independently selected from $C_6$ to $C_{24}$ aryl, $C_1$ to $C_{20}$ alkyl, phosphine oxide group and CN and the heteroatom is selected from O, S, Se, N, alternatively O, S, Se; and
$R_3$ is selected from unsubstituted or substituted $C_6$ to $C_{24}$ aryl, wherein the substituents are selected from $C_6$ to $C_{24}$ aryl, $C_1$ to $C_{20}$ alkyl, phosphine oxide group and CN.

**11.** Compound according to claim 10, wherein all $Y^4$ to $Y^6$ are N.

**12.** Compound according to any of the claims 10 or 11, wherein G is selected from the group consisting of dibenzofurane, benzofurane, dibenzothiophene and benzothiophene.

**13.** Compound according to any of the claims 10 to 12, wherein $R^3$ is phenyl.

**Patentansprüche**

**1.** Organische lichtemittierende Vorrichtung, umfassend eine Kathode (190), eine Anode (120), eine lichtemittierende Schicht (150, 151), mindestens eine erste Elektronentransportschicht und mindestens eine zweite Elektronentransportschicht, wobei die lichtemittierende Schicht, die erste Elektronentransportschicht (156) und die zweite Elektronentransportschicht (161) zwischen der Kathode und der Anode angeordnet sind,

wobei die erste Elektronentransportschicht eine Verbindung der Formel (I) umfasst,

**L-M**

wobei in Formel (I)
L ausgewählt ist aus unsubstituiertem oder substituiertem $C_6$- bis $C_{60}$-Aryl, wobei der eine oder die mehreren Substituenten, falls vorhanden, unabhängig ausgewählt sind aus $C_2$- bis $C_{24}$-Alkenyl, $C_1$- bis $C_{20}$-Alkyl, $C_7$- bis $C_{32}$-Arylalkyl, $C_7$- bis $C_{32}$-Arylalkenyl; M substituiertes Pyrimidin oder substituiertes Triazin ist, wobei der eine oder die mehreren Substituenten unabhängig ausgewählt sind aus $C_6$- bis $C_{24}$-Aryl, wobei das $C_6$-bis $C_{24}$-Aryl unsubstituiert oder mit $C_1$- bis $C_3$-Alkyl substituiert sein kann;
L und M über eine direkte Bindung oder über eine Spacereinheit, ausgewählt aus nicht kondensiertem $C_6$- bis $C_{18}$-Aryl, das unsubstituiert oder substituiert sein kann, verbunden sein können, wobei der eine oder die mehreren Substituenten, falls vorhanden, unabhängig ausgewählt sind aus $C_6$- bis $C_{12}$-Aryl, $C_2$- bis $C_{20}$-Heteroaryl und Phosphinoxidgruppe; und
die Verbindung der Formel (I) ein molekulares Dipolmoment von $\leq$ 5 Debye aufweist;
wobei die zweite Elektronentransportschicht eine Verbindung der Formel (II) umfasst,

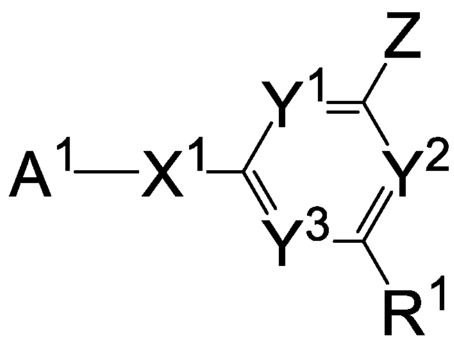

wobei in Formel (II)
$A^1$ ausgewählt ist aus unsubstituiertem oder substituiertem $C_{10}$- bis $C_{24}$-Aryl, umfassend mindestens ein aromatisches Ringsystem, umfassend mindestens zwei kondensierte 6-gliedrige aromatische Ringe, wobei der eine oder die mehreren Substituenten, falls vorhanden, unabhängig ausgewählt sind aus $C_6$- bis $C_{24}$-Aryl, $C_2$- bis $C_{20}$-Heteroaryl und diese Substituenten ferner mit einer Phosphinoxidgruppe substituiert sein können;
$X^1$ ausgewählt ist aus unsubstituiertem oder substituiertem $C_6$- bis $C_{24}$-Aryl, wobei der eine oder die mehreren Substituenten, falls vorhanden, unabhängig ausgewählt sind aus $C_6$- bis $C_{12}$-Aryl, $C_2$- bis $C_{10}$-Heteroaryl und diese Substituenten ferner mit einer Phosphinoxidgruppe oder CN substituiert sein können;
$Y^1$ bis $Y^3$ unabhängig ausgewählt sind aus N und $CR_2$, vorausgesetzt, dass mindestens zwei von $Y^1$ bis $Y^3$ N sind, wobei $R^2$ ausgewählt ist aus H oder $C_1$- bis $C_3$-Alkyl;
Z ausgewählt ist aus unsubstituiertem oder substituiertem $C_6$- bis $C_{24}$-Aryl oder unsubstituiertem oder substituiertem $C_8$- bis $C_{24}$-Heteroaryl, wobei der eine oder die mehreren Substituenten, falls vorhanden, unabhängig ausgewählt sind aus $C_6$- bis $C_{24}$-Aryl, $C_1$- bis $C_{20}$-Alkyl, Phosphinoxidgruppe und CN; und
R1 ausgewählt ist aus unsubstituiertem oder substituiertem $C_6$- bis $C_{24}$-Aryl, wobei der eine oder die mehreren Substituenten, falls vorhanden, unabhängig ausgewählt sind aus $C_6$- bis $C_{24}$-Aryl, $C_1$- bis $C_{20}$-Alkyl, Phosphinoxidgruppe und CN.

2. Organische lichtemittierende Vorrichtung nach Anspruch 1, wobei L unsubstituiertes oder substituiertes Fluoren oder Phenylen ist, wobei das Phenylen mit 1 bis 5 Phenylgruppen substituiert ist.

3. Organische lichtemittierende Vorrichtung nach Anspruch 1 oder 2, wobei M substituiertes Triazin ist, wobei der eine oder die mehreren Substituenten unabhängig ausgewählt sind aus $C_6$- bis $C_{24}$-Aryl, das unsubstituiert oder mit $C_1$- bis $C_3$-Alkyl substituiert sein kann.

4. Organische lichtemittierende Vorrichtung nach einem der vorhergehenden Ansprüche, wobei die Spacereinheit Phenylen oder Biphenylen ist.

5. Organische lichtemittierende Vorrichtung nach einem der vorhergehenden Ansprüche, wobei A1 substituiertes Anthracen ist, wobei der eine oder die mehreren Substituenten unabhängig ausgewählt sind aus $C_6$- bis $C_{24}$-Aryl und $C_2$- bis $C_{20}$-Heteroaryl und diese Substituenten ferner mit einer Phosphinoxidgruppe oder CN substituiert sein können.

**6.** Organische lichtemittierende Vorrichtung nach einem der vorhergehenden Ansprüche, wobei $X^1$ Phenylen ist.

**7.** Organische lichtemittierende Vorrichtung nach einem der vorhergehenden Ansprüche, wobei alle $Y^1$ bis $Y^3$ N sind.

**8.** Organische lichtemittierende Vorrichtung nach einem der vorhergehenden Ansprüche, wobei Z ausgewählt ist aus unsubstituiertem oder substituiertem $C_6$- bis $C_{24}$-Aryl.

**9.** Organische lichtemittierende Vorrichtung nach einem der vorhergehenden Ansprüche, wobei die mindestens eine zweite Elektronentransportschicht ein Metall, ein Metallsalz oder einen Metallkomplex umfasst.

**10.** Verbindung mit der allgemeinen Formel (III)

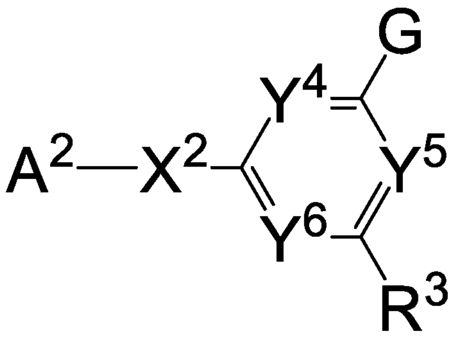

wobei

$A^2$ substituiertes Anthracen oder substituiertes $C_{10}$-Aryl ist, umfassend mindestens zwei kondensierte 6-gliedrige aromatische Ringe, wobei der eine oder die mehreren Substituenten unabhängig ausgewählt sind aus $C_6$- bis $C_{24}$-Aryl und $C_2$- bis $C_{20}$-Heteroaryl und diese Substituenten ferner mit einer Phosphinoxidgruppe oder CN substituiert sein können;
$X^2$ Phenylen ist;
$Y^4$ bis $Y^6$ unabhängig ausgewählt sind aus N und $CR^4$, vorausgesetzt, dass mindestens zwei von $Y^4$ bis $Y^6$ N sind, wobei $R^4$ ausgewählt ist aus H oder $C_1$- bis $C_3$-Alkyl;
G ausgewählt ist aus unsubstituiertem oder substituiertem $C_8$- bis $C_{24}$-Heteroaryl, umfassend mindestens einen 5-gliedrigen Ring, wobei der eine oder die mehreren Substituenten, falls vorhanden, unabhängig ausgewählt sind aus $C_6$- bis $C_{24}$-Aryl, $C_1$- bis $C_{20}$-Alkyl, Phosphinoxidgruppe und CN und das Heteroatom ausgewählt ist aus O, S, Se, N, alternativ O, S, Se; und
R3 ausgewählt ist aus unsubstituiertem oder substituiertem $C_6$- bis $C_{24}$-Aryl, wobei die Substituenten ausgewählt sind aus $C_6$- bis $C_{24}$-Aryl, $C_1$- bis $C_{20}$-Alkyl, Phosphinoxidgruppe und CN.

**11.** Verbindung nach Anspruch 10, wobei alle $Y^4$ bis $Y^6$ N sind.

**12.** Verbindung nach einem der Ansprüche 10 oder 11, wobei G ausgewählt ist aus der Gruppe bestehend aus Dibenzofuran, Benzofuran, Dibenzothiophen und Benzothiophen.

**13.** Verbindung nach einem der Ansprüche 10 bis 12, wobei $R^3$ Phenyl ist.

## Revendications

**1.** Dispositif émetteur de lumière organique comprenant une cathode (190), une anode (120), une couche émettrice de lumière (150, 151), au moins une première couche de transport d'électrons et au moins une deuxième couche de transport d'électrons, la couche électroluminescente, la première couche de transport d'électrons (156) et la deuxième couche de transport d'électrons (161) sont disposées entre la cathode et l'anode, la première couche de transport d'électrons comprenant un composé de formule (I)

**L-M**

dans lequel dans la formule (I)

L est sélectionné parmi les groupes aryle non substitués ou substitués en $C_6$ à $C_{60}$, le ou les substituants, s'ils sont présents, étant indépendamment sélectionnés parmi alcényle en $C_2$ à $C_{24}$, alkyle en $C_1$ à $C_{20}$, aryl-alkyle en $C_7$ à $C_{32}$, aryl-alcényle en $C_7$ à $C_{32}$ ;
M est une pyrimidine substituée ou une triazine substituée, dans lequel le ou les substituants sont sélectionnés indépendamment entre un groupe aryle en $C_6$ à $C_{24}$ et dans lequel le groupe aryle en $C_6$ à $C_{24}$ peut être non substitué ou substitué par un alkyle en $C_1$ à $C_3$ ;
L et M peuvent être connectés via une liaison directe ou via un fragment espaceur sélectionné parmi un aryle non condensé en $C_6$ à $C_{18}$ qui peut être non substitué ou substitué, dans lequel le ou les substituants, s'ils sont présents, sont indépendamment sélectionnés parmi un aryle en $C_6$ à $C_{12}$, hétéroaryle en $C_2$ à $C_{20}$ et un groupe oxyde de phosphine ; et
le composé de formule (I) a un moment dipolaire moléculaire de ≤ 5 Debye ;
dans lequel la deuxième couche de transport d'électrons comprend un composé de formule

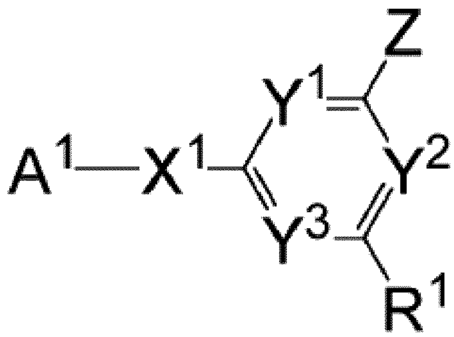

dans lequel dans la formule (II)
$A^1$ est sélectionné parmi un aryle $C_{10}$ à $C_{24}$ non substitué ou substitué comprenant au moins un système de noyaux aromatiques comprenant au moins deux noyaux aromatiques condensés à 6 chaînons, le ou les substituants, s'ils sont présents, étant indépendamment sélectionnés parmi un aryle en $C_6$ à $C_{24}$, un hétéroaryle en $C_2$ à $C_{20}$ et ces substituants peuvent en outre être substitués par un groupe oxyde de phosphine ;
$X^1$ est sélectionné parmi un aryle en $C_6$ à $C_{24}$ non substitué ou substitué, le ou les substituants, s'ils sont présents, étant indépendamment sélectionnés parmi un aryle en $C_6$ à $C_{12}$, un hétéroaryle en $C_2$ à $C_{10}$ et ces substituants peuvent en outre être substitués par un groupe oxyde de phosphine ou CN ;
$Y^1$ à $Y^3$ sont sélectionnés indépendamment parmi N et $CR^2$ à condition qu'au moins deux des $Y^1$ à $Y^3$ représentent N, dans lequel le $R^2$ est sélectionné parmi H ou un alkyle en $C_1$ à $C_3$ ;
Z est sélectionné parmi un aryle en $C_6$ à $C_{24}$ non substitué ou substitué ou un hétéroaryle en $C_8$ à $C_{24}$ non substitué ou substitué, dans lequel le ou les substituants, s'ils sont présents, sont indépendamment sélectionnés parmi un aryle en $C_6$ à $C_{24}$, un alkyle en $C_1$ à $C_{20}$, un groupe oxyde de phosphine et CN ; et
$R^1$ est sélectionné parmi un aryle en $C_6$ à $C_{24}$ non substitué ou substitué, dans lequel le ou les substituants, s'ils sont présents, sont indépendamment sélectionnés parmi un aryle en $C_6$ à $C_{24}$, un alkyle en $C_1$ à $C_{20}$, un groupe oxyde de phosphine et CN.

2. Dispositif électroluminescent organique selon la revendication 1, dans lequel L est un fluorène ou un phénylène non substitué ou substitué, le phénylène étant substitué par 1 à 5 groupes phényle.

3. Dispositif électroluminescent organique selon la revendication 1 ou 2, dans lequel M est une triazine substituée dans lequel le ou les substituants sont sélectionnés indépendamment parmi un aryle en $C_6$ à $C_{24}$ qui peut être non substitué ou substitué par un alkyle en $C_1$ à $C_3$.

4. Dispositif électroluminescent organique selon une quelconque des revendications précédentes, dans lequel le fragment spatial est le phénylène ou le biphénylène.

5. Dispositif électroluminescent organique selon une quelconque des revendications précédentes, dans lequel $A^1$ est un anthracène substitué, dans lequel le ou les substituants sont sélectionnés indépendamment parmi un aryle en $C_6$ à $C_{24}$ et un hétéroaryle en $C_2$ à $C_{20}$ et ces substituants peuvent en outre être substitués par un groupe oxyde phosphine ou CN.

**6.** Dispositif électroluminescent organique selon une quelconque des revendications précédentes, dans lequel $X^1$ est le phénylène.

**7.** Dispositif électroluminescent organique selon une quelconque des revendications précédentes, dans lequel tous $Y^1$ à $Y^3$ sont N.

**8.** Dispositif électroluminescent organique selon une quelconque des revendications précédentes, dans lequel Z est sélectionné parmi un groupe aryle en $C_6$ à $C_{24}$ non substitué ou substitué.

**9.** Dispositif électroluminescent organique selon une quelconque des revendications précédentes, dans lequel la au moins une deuxième couche de transport d'électrons comprend un métal, un sel métallique ou un complexe métallique.

**10.** Composé ayant la formule générale (III)

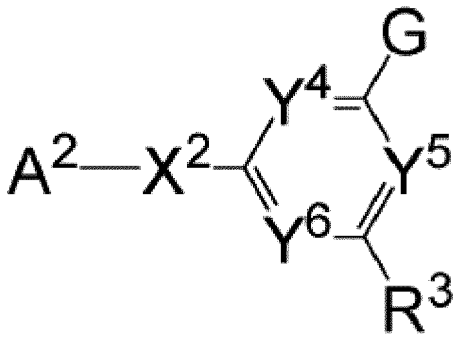

dans lequel

$A^2$ représente un anthracène substitué ou un aryle $C_{10}$ substitué comprenant au moins deux cycles aromatiques condensés à 6 chaînons, dans lequel le ou les substituants sont sélectionnés indépendamment parmi un aryle en $C_6$ à $C_{24}$ et un hétéroaryle en $C_2$ à $C_{20}$ et ces substituants peuvent en outre être substitués par un groupe oxyde de phosphine ou CN;
$X^2$ est un phénylène ;
$Y^4$ à $Y^6$ sont sélectionnés indépendamment parmi N et $CR^4$ à condition qu'au moins deux des $Y^4$ à $Y^6$ sont N, dans lequel $R^4$ est sélectionné parmi H ou un alkyle en $C_1$ à $C_{3;}$
G est sélectionné parmi un hétéroaryle en $C_8$ à $C_{24}$ non substitué ou substitué comprenant au moins un cycle à 5 chaînons, dans lequel le ou les substituants, s'ils sont présents, sont indépendamment sélectionnés parmi un aryle en $C_6$ à $C_{24}$, un alkyle en $C_1$ à $C_{20}$, un groupe oxyde de phosphine et CN et l'hétéroatome est sélectionné parmi O, S, Se, N, ou encore O, S, Se ; et
$R^3$ est sélectionné parmi un aryle en $C_6$ à $C_{24}$ non substitué ou substitué, dans lequel les substituants étant sélectionnés entre un aryle en $C_6$ à $C_{24}$, un alkyle en $C_1$ à $C_{20}$, un groupe oxyde de phosphine et CN.

**11.** Composé selon la revendication 10, dans lequel tous les $Y^4$ à $Y^6$ sont N.

**12.** Composé selon une quelconque des revendications 10 ou 11, dans lequel G est sélectionné dans le groupe constitué du dibenzofurane, du benzofurane, du dibenzothiophène et du benzothiophène.

**13.** Composé selon une quelconque des revendications 10 à 12, dans lequel $R^3$ est un phényle.

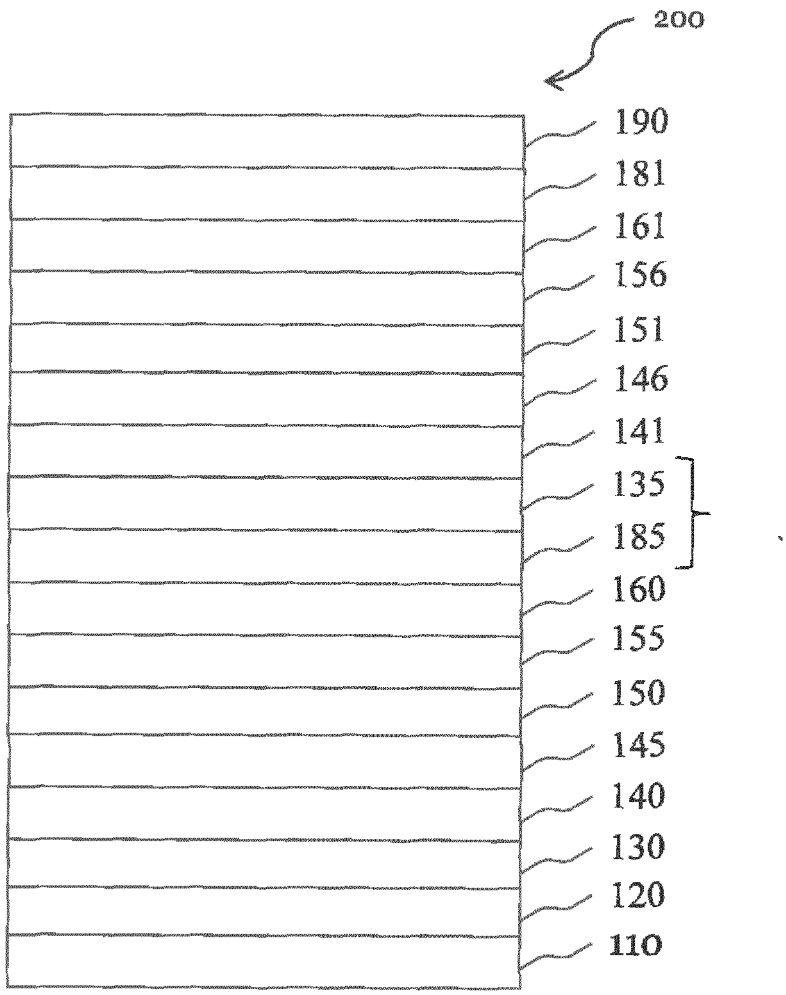
200
190
181
161
156
151
146
141
135
185
160
155
150
145
140
130
120
110

Fig.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2018139662 A
- EP 3373353 A
- US 2009212280 A
- US 2007252140 A
- EP 2722908 A1
- EP 1970371 A1
- WO 2013079217 A1
- US 2016322581 A
- WO 2015174640 A
- US 2008265216 A
- WO 2016171358 A
- WO 2016105141 A
- WO 2013079217 A

### Non-patent literature cited in the description

- *CHEMICAL ABSTRACTS,* 14728-62-2
- *CHEMICAL ABSTRACTS,* 1440864-50-5
- *CHEMICAL ABSTRACTS,* 51870-56-5
- *CHEMICAL ABSTRACTS,* 1623748-16-2
- **YASUHIKO SHIROTA ; HIROSHI KAGEYAMA.** *Chem. Rev.,* 2007, vol. 107, 953-1010
- *CHEMICAL ABSTRACTS,* 1242056-42-3
- *CHEMICAL ABSTRACTS,* 1824678-59-2
- *CHEMICAL ABSTRACTS,* 1224447-88-4
- *CHEMICAL ABSTRACTS,* 2032364-64-8
- *CHEMICAL ABSTRACTS,* 1955543-57-3
- *CHEMICAL ABSTRACTS,* 850918-68-2